# EUROPEAN PATENT SPECIFICATION

(11) **EP 0 386 713 B1**
(45) Date of publication and mention of the grant of the patent: **18.09.1996**
(21) Application number: 90104301.8
(22) Date of filing: 07.03.1990
(51) Int. Cl.: G01N 33/569, C12N 15/49

(54) **Immunoassay for antibodies to HIV**
Immunoassay zum Nachweis von Antikörpern gegen HIV
Essai immunologique des anticorps contre HIV

(30) Priority: 09.03.1989 US 320882; 02.06.1989 US 361733
(43) Date of publication of application: 12.09.1990
(73) Proprietor: ABBOTT LABORATORIES, Abbott Park, Illinois 60064-3500 (US)
(72) Inventor: Dawson, George J., Libertyville, IL 60048 (US); Mimms, Larry T., Lake Villa, IL 60046 (US); Wood, Charles, Lawrence, KS 66044 (US); Wu, Ping, Highland Park, IL 60035 (US); Roser, Michelle A., Chicago, IL 60614 (US)
(74) Representative: Modiano, Guido, Dr.-Ing.

(56) References cited:
- EP-A- 0 226 903
- EP-A- 0 265 851
- WO-A-89/01527
- PROCEEDINGS OF THE NATL. ACADEMY OF SCIENCES USA, vol. 82, no. 22, November 1985, Washington, DC (US); DOWBENKO et al., pp. 7748-7752
- BIOTECHNOLOGY, vol. 4, February 1986, New York, NY (US); CABRADILLA et al., pp. 128-133
- KEMENY and CHALLACOMBE (Eds): "ELISA and other solid phase immunoassays", 1988, John Wiley & Sons Ltd., Colchester, England, pp. 1-29

## Description

### BACKGROUND OF THE INVENTION

The present invention relates in general to an improved assay for detection, in human sera, of antibodies to the viral causative agent of acquired immunodeficiency syndrome, and in particular to a specific and sensitive method for detection of antibodies to the human immunodeficiency virus (HIV) using safe, bacterially-produced recombinant HIV proteins.

Acquired immunodeficiency syndrome (AIDS), which has reached epidemic proportion in the United States, Europe and Central Africa, is a disorder of the immune system associated with opportunistic infections and/or neoplasms. Among the challenges to be faced is the protection of blood products from contamination by the causative agent of AIDS, human immunodeficiency virus (HIV), variants of which include viruses which are commonly referred to as human T-cell lymphotropic virus type III (HTLV-III), AIDS-related virus (ARV), lymphadenopathy-associated virus (LAV), etc.

Based on the nucleotide analysis of the viral genome, beginning at the 5' end, the HIV genomic RNA encodes:
(i) a gag gene extending between nucleotides 310 to 1,869 and encoding for the internal structural core or nucleocapsid proteins including p24, the most antigenic core protein;
(ii) a pol gene extending between nucleotides 1,629 to 4,673 and encoding for the enzyme, reverse transcriptase; and
(iii) an env gene extending between nucleotides 5,781 to 8,369 and encoding for the envelope glycoproteins including gp41, the most antigenic envelope protein;
Ratner et al, Nature, 313, 277-284 (1985).

At the time HIV was isolated from cultured T-cells of patients with AIDS and determined to be the probable causative agent, a search was launched for an assay method that would be useful as a diagnostic indicator of the disease and also useful for screening blood products. Immunoassays, because of their sensitivity, were the likely choice.

One immunoassay approach, Gallo et al, U.S. Patent No. 4,520,113, describes three assays for anti-HIV. They comprise a strip radioimmunoassay based on the Western Blot technique, an enzyme-linked immunosorbent assay (ELISA), and an indirect immunofluorescence assay. The viral protein reagent used in these immunoassays consists of inactivated whole virus isolated from cell cultures of the immortalized neoplastic human T-cell line, HT.

Current immunoassays designed to detect the presence of antibodies to the HIV virus in human sera use an ELISA method employing inactivated whole virus cultured in a cell line capable of virus replication as the antigen reagent. This method is quite sensitive, and there is a high likelihood that a truly infected person will be detected. However, uninfected persons may occasionally give positive results.

The detection of false positives in screening assays of blood products is one of the most pressing problems with the commonly used methods. Presently, specimen samples are screened by an ELISA method and if the result is positive, two more tests are run on the sample. If either is positive, the specimen is said to be repeatedly reactive. The Western Blot is the method commonly used to determine whether repeatedly reactive specimens contain antibodies to HIV. However, low value repeatedly reactive specimens often do not give a positive result on the Western Blot. Dassey et al., JAMA, 255, 743-745 (1986). Nevertheless, the blood product must be disposed of because doubt exists regarding its infectivity. Chalmers et al, JAMA, 256, 1778- 1783 (1986). These false positives are often due to nonspecific binding of immunoglobulins to cellular protein in the viral isolates.

Another area of concern with the presently available assays is the lack of a convenient confirmatory assay to help resolve false positives. The currently used procedure, the Western Blot, generally described by Towbin et al., Proc. Nat'l Acad. Sci. (USA), 76, 4350 (1979), requires sodium dodecylsulfate polyacrylamide gel electrophoresis (SDS PAGE) to separate HIV proteins based on their molecular weight. The separated HIV proteins are transferred electrophoretically from the gel to a nitrocellulose sheet. The nitrocellulose sheet is cut into strips and reacted with the test sample and then with an anti-human IgG labeled antibody. A positive result is the appearance of label at the area on the strip to which the viral proteins have migrated. Among the disadvantages of the procedure for detecting anti-HIV are the requirements for subjective interpretation, the complexity and laboriousness associated with the techniques, and the inherent difficulty in controlling variations in the composition of HIV proteins.

The report of a positive anti-HIV assay can be devastating to a patient. Further, a false positive in blood product specimens is undesirable because the donated blood must be destroyed. Therefore, the elimination of false positives is a worthwhile endeavor.

Safety is another concern associated with the present assay method because the procedures entail culturing live virus in vitro with subsequent isolation and deactivation to provide the whole virus reagent. Both the culturing and isolation processes are inherently dangerous since HIV infection is potentially fatal.

Although the most specific test for HIV infection remains virus isolation, because of the complexity and difficulty of isolating HIV in culture, this is an impractical method for large scale use. Some form of the ELISA method with enhanced sensitivity and specificity remains the most practical and feasible procedure for large-scale screening of the blood supply.

The use of recombinantly-produced HIV protein as an antigen reagent in assay methods may solve the aforementioned problems. First, recombinant proteins are primarily non-infectious and therefore production and isolation of such proteins would be safer than the culturing of whole virus. Secondly, the use of pure viral protein obtained by recombinant methods should eliminate some of the false positives due to nonspecific reactions with contaminating proteins. Thirdly, standardization of reagents should improve the specificity and predictive value of the assay.

Expression of HIV gag gene proteins, including p24, in E. coli have indicated that the HIV gag proteins produced by rDNA technology could have potential diagnostic value. Wood et al., Cold spring Harbor Symposium on RNA Tumor Viruses, Cold Spring Harbor, New York, May 22-26, 1985; Dowbekno et al., Proc. Natl. Acad. Sci. U.S.A., 82, 7748-7752, (1985); Ghrayeb et al., DNA, 5, 93-39 (1986); Steimer et al, Virology, 150, 283-290 (1986). Similarly, the expression of gp41 or parts of gp41 have also demonstrated the utility of rDNA derived HIV envelope sequences in diagnostic assays. Wood et al., Cold Spring Harbor Symposium on RNA Tumor Viruses, Cold Spring Harbor, New York, May 22-26, 1985; Chang et al., Biotechnology, 3, 905-909 (1985); Crowl et al. Cell, 41, 979-986 (1985); Cabradilla et al., Biotechnology, 4, 128-133 (1986). While it is general knowledge that viral proteins expressed in E. coli have potential utility in diagnostic assays, development of immunoassays using these reagents which will have the specificity and sensitivity equal to or greater than the native viral proteins derived from the cell culture has been a difficult task.

Of interest as background to the present invention is EP-A-226 903 which discloses a competitive assay method for detecting antibody to HTLV-III-p24 and HTLV-III-gp41 in a biological sample utilizing recombinant p24 and gp41 attached to a single or separate solid supports and further utilizing labeled anti-HTLV-III-p24 and labeled anti-HTLV-III-gp41.

Also of interest is EP-A-265 851 which discloses a competitive assay method for detecting antibody to HIV using a solid support whereon p24 and gp41 are attached mixed or at separate parts of the support. The support is contacted with the biological sample and labeled antibodies.

### SUMMARY OF THE INVENTION

The present invention provides an improved immunoassay method for detection of antibodies to HIV in a biological sample said method comprising at least two detection systems, one for the detection of antibodies to HIV-p24 and another for the detection of HIV-gp41.

One method according to the invention comprises the steps of:
(a) contacting a first aliquot of a biological sample with a first solid support to which HIV-1 p24 recombinant protein has been attached;
(b) contacting the first solid support with labeled HIV-1 p24 recombinant protein;
(c) detecting the label to determine the presence of anti-HIV-1 p24 in the biological sample;
(d) contacting a second aliquot of the biological sample with a second solid support to which HIV-1 gp41 recombinant protein has been attached;
(e) contacting the second solid support with labeled HIV-1 gp41 recombinant protein;
(f) detecting the label to determine the presence of anti-HIV-1 gp41 in the biological sample;
wherein the presence of either anti-HIV-1 p24 or anti-HIV-1 gp41, or both, indicates the presence of anti-HIV in the biological sample.

In one embodiment of such method, steps a and b are performed simultaneously and steps d and e are performed simultaneously.

Another method according to the invention comprises the steps of:
(a) contacting a biological sample with a solid support to which HIV-1 p24 recombinant protein and HIV-1 gp41 recombinant protein have been attached;
(b) contacting the solid support with labeled HIV-1 p24 recombinant protein and labeled HIV-1 gp41 recombinant protein;
(c) detecting the label to determine the presence of anti-HIV-1 p24 or anti-HIV-1 gp41, or both, in the biological sample;
wherein the presence of either anti-HIV-1 p24 or anti-HIV-1 gp41, or both, indicates the presence of anti-HIV in the biological sample.

In one embodiment of such method, steps a and b are performed simultaneously.

Another embodiment according to the invention comprises the steps of:
(a) contacting a first aliquot of a biological sample with a first solid support to which HIV-1 p24 recombinant protein has been attached;
(b) contacting the first solid support with HIV-1 p24 recombinant protein conjugated to a first hapten;
(c) contacting the first solid support with labeled antibody to the first hapten;
(d) detecting the label to determine the presence of anti-HIV-1 p24 in the biological sample;
(e) contacting a second aliquot of the biological sample with a second solid support to which HIV-1 gp41 recombinant protein has been attached;
(f) contacting the second solid support with HIV-1 gp41 recombinant protein conjugated to a second hapten;
(g) contacting the second solid support with labeled antibody to the second hapten;
(h) detecting the label to determine the presence of anti-HIV-1 gp41 in the biological sample;
wherein the presence of either anti-HIV-1 p24 or anti-HIV-1 gp41, or both, indicates the presence of anti-HIV in the biological sample.

Another embodiment according to the invention comprises the steps of:
(a) contacting a biological sample with a solid support to which HIV-1 p24 recombinant protein and HIV-1 gp41 recombinant protein have been attached;
(b) contacting the solid support with HIV-1 p24 recombinant protein conjugated to a first hapten and HIV-1 gp41 recombinant protein conjugated to a second hapten;
(c) contacting the solid support with labeled antibody to the first and second haptens;
(d) detecting the label to determine the presence of anti-HIV-1 p24 or anti-HIV-1 gp41, or both, in the biological sample;
wherein the presence of either anti-HIV-1 p24 or anti-HIV-1 gp41, or both, indicates the presence of anti-HIV in the biological sample.

Biological samples which are easily tested by the methods of the present invention include human and animal body fluids such as whole blood, serum, plasma, urine, saliva, stools, lymphocyte or cell culture preparations and purified and partially purified immunoglobulins. Solid supports which can be used in the immunoassay of the invention include wells of reaction trays, test tubes, beads, strips, membranes, filters, microparticles or other solid supports which are well known to those skilled in the art. Enzymatic, radioisotopic, fluorescent and chemiluminescent labels can be used in the aforementioned assay. Further, hapten/labeled anti-hapten systems such as a biotin/labeled anti-biotin system may be utilized in the inventive assay. Both polyclonal and monoclonal antibodies are useful as reagents, and IgG as well as IgM class HIV antibodies may be used as solid support or labeled reagents.

### BRIEF DESCRIPTION OF THE FIGURES

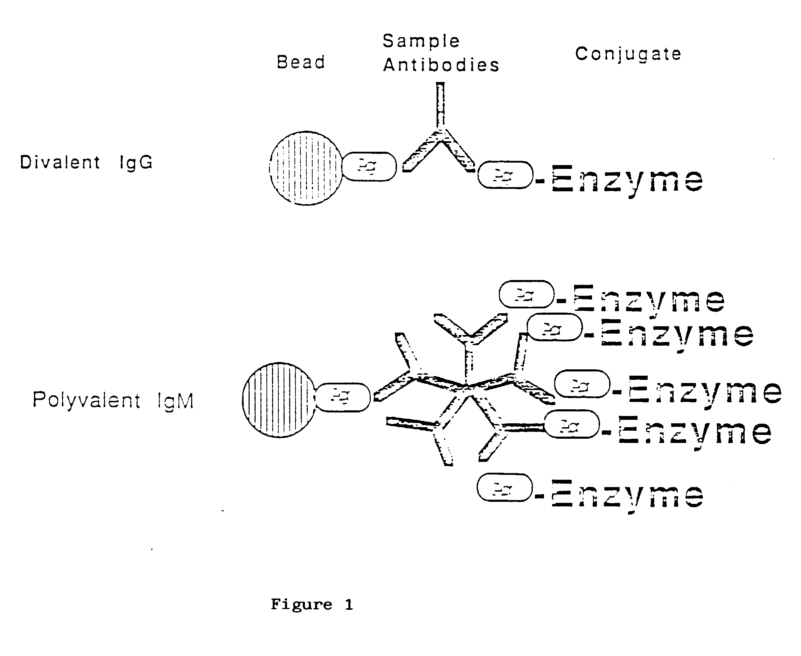

- Figure 1: compares the detectability of decavalent IgM in the sandwich format (bottom) with IgG (top) Note the potential of decavalent IgM to bind more antigen conjugate than divalent IgG allowing each captured immunoglobulin M molecule to generate more signal than each captured IgG molecule.

### DETAILED DESCRIPTION OF THE INVENTION

The present invention is a dual immunoassay using both envelope and core bacterially-produced proteins as assay reagents. HIV-infected HT-9 cells were harvested and total cellular DNA was isolated and subjected to digestion. The DNA fragments produced were molecularly cloned and were selected for HIV genomic sequences. The DNA segments encoding for the core protein and for the envelope glycoprotein were further subcloned into bacterial expression vectors using well-known recombinant technology. The bacterially-expressed proteins were used as antigen reagents to assay patient sera for the presence of antibodies specifically reactive with HIV.

Following infection with HIV-1, virus antigens can be detected in patient plasma for a period of time that varies between individuals. Over time after infection, the titer of antigen decreases simultaneously as antibodies to HIV-1 develop. The sensitivity of previous anti-HIV-1 assays is such that there is a period of time between infection and the detection of antibodies (seroconversion). Measurement of circulating HIV-1 antigen levels, while shortening this period, does not completely close this interval. Thus it is possible for an individual infected with HIV-1 to have both undetectable HIV antigen and HIV-1 antibodies for a critical period of time after infection.

The assay format of the inventi-on allows for earlier detection of HIV-1 antibodies than previous anti-HIV assays. In the case of polymeric IgM this format allows the capture of multiple antigen conjugate molecules by a single bound IgM molecule resulting in more bound enzyme and increased sensitivity to IgM (Figure 1). IgM has been observed to be a major component of the initial response to infection. In addition the double specificity requirement for reactivity in the assay allows the use of a larger volume than is utilized by conventional enzyme immunoassays (EIAs) without compromising specificity.

The fundemental principles of this assay format utilize antigens coated on to the solid-phase to bind specifically to antibodies present in patient serum. These antibodies then specifically bind to antigens conjugated to an enzyme. The specificity of the antibody antigen interaction is necessary at both stages of the overall reaction unlike conventional EIA where specifically bound antibodies are detected by an enzyme labelled antiIgG antibodies which recognize any bound IgG.

In 34 of 36 seroconversion samples the Example 10 assay detects HIV-1 antibodies with higher signal and cutoff (S/CO) than the previous anti-HIV tests. The Example 10 assay detected all 36 seroconversion samples earlier than the conventional viral-lysate assay. Additionally, several samples are detected as positive by Example 10 assay which test negative with one or both of the previous anti-HiV assays. For example, in the case of panel 2 the assay of Example 10 detects as positive a sample taken on day 6 whereas the assay of Example 7 only detects the sample taken on day 8 as positive. The viral-lysate based assay is borderline at day 14 and positive at day 21.

In panel 2 a relatively high level of IgM is observed early in the series with IgG rising later. The pattern of signal obtained from the assay of Example 10, a rapid rise with subsequent fall to a plateau, is in sharp contrast to the previous anti-HIV assay signals which rise steadily as time progresses.

Panel 4 covers a later period in seroconversion as IgM levels have begun to decline. The signal from the assay of Example 10 drops as IgM levels fall and competing IgG levels rise until IgG rises sufficiently to replace the IgM signal with IgG derived signal whereupon the inventive signal rises in parallel with IgG levels.

The Example 10 and Example 7 signals rise in parallel in panel 1. This panel has has relatively low initial IgM levels and high early IgG levels.

The biphasic response seen uniquely in the Example 10 assay in panels 2,5, and 7 indicates that this assay detects IgM with greater efficiency than IgG because of pentmeric IgM captured by the bead binding more than one conjugate molecule. Early in seroconversion if substantial IgM is present without much competing IgG the multivalent IgM results in high signals in the inventive format. As IgM levels fall, and as competing IgG levels rise the divalent IgG competes with decavalent IgM and-is-detected less efficiently. Thus, the total signal observed in the inventive assay declines. Finally, as IgG levels continue to rise and IgM levels continue to fail the total signal from the assay rises again to form the second phase of the response.

In contrast, the signals observed with the previous anti-HIV assays rises steadily in all panels except =2, with no biphasic response. Both these assays utilise HRPO conjugated to anti-human IgG heavy and light chain raised in goats to detect captured antibodies. This lack of early response indicates that either these previous anti-HIV assays do not detect IgM in these seroconversion panels or that they do detect IgM by virtue of heavy chain cross-reactivity but no more efficiently than IgG.

Thus, Example 10 demonstrates an enzyme immunoassay format that allows for earlier detection of HIV antibody because of increased sensitivity to decavalent immunoglobulin M (IgM).

### EXAMPLE 1

### Cloning of the HIV Genome and Expression of Core and Envelope Protein in E. coli

Using density gradient centrifugation, HIV virus was purified from culture fluids of HIV-infected HT-9 cells supplied by Frederick Cancer Research Facility, Frederick, Maryland. A viral. cDNA library was constructed using purified viral RNA extracted from banded virus. Okayama et al., Mol. and Cell. Biol., 3, 280-289 (1983).

Labeled viral CDNA fragments were used as probes to screen a cDNA library constructed from poly A selected viral RNA. One clone, pCW11, containing the entire viral 3′ LTR plus the 3′ open reading frame was used as a probe for subsequent screening of the genomic library resulting in isolation of several clones containing partial or entire viral genome. Using these clones, expression vectors for production of p24 and gp41 in E. coli were constructed.

One clone, pC23, containing the entire viral gag gene encoded 3 core proteins of about 17 kD (p17), 24 kD (p24) and 15 kD (p15). The most antigenic of the three, p24 was chosen for expression in E. coli. The p24 gene fragment was inserted into plasmid, pUC-9 (Pharmacia, Piscataway, New Jersey) at a position that would render it under the control of the lac promoter. The resultant plasmid, designated pB1 included DNA encoding for 13 amino acids at the carboxyl terminus of the p17 protein, the entire p24 protein and 59 amino acids of the p15 protein. The protein expressed in E. coli matched well with the expected molecular weight and could be readily detected by Western Blot analysis.

Another clone, p41C, consisting of an 845 bp fragment of the envelope gene, flanked by Bgl II and Kpn I restriction sites encoded the carboxyl terminus of gp120 (45 amino acids) and the entire gp41 protein. Insert ion of this gene fragment into pUC-9 and expression were performed as in the above-mentioned p24 example. Western Blot analysis confirmed the expected molecular weight and antigenicity.

Host cells used for propagation of gp41 and p24 were E. coli K-12, strain JM103, ( (lac-pro), sup E, thi, str A, sbc B15, end A, hsd R4/F'tra D 36, pro AB, lac I^{q} Z M15). Messing et al., Methods Enymol., 101, 20-78 (1983). Vectors containing the lac Z gene are commercially available (Pharmacia, Piscataway, New Jersey) . All manipulations involving nucleic acids have been described in Maniatis et al., Molecular Cloning, Cold Spring Harbor Laboratory, 3-17 (1982).

### EXAMPLE 2

### Purification and Characterization of gp41 and P24

The HIV gp41 recombinant protein produced in E. coli (clone p41C) was purified using affinity column and ion exchange chromatography. The bacterial lysate supernatant was passed over an affinity column composed of Sepharose® 4B beads bound with monoclonal anti-HIV-gp41. The column was washed with a buffer of 0.5%- TRITON® X-100 and the bound HIV gp41 was eluted with the same buffer containing 5 M NaI. The eluted protein solution was dialyzed extensively to remove NaI and mixed 1:1 with an ethanolamine buffer containing 0.1% Tween® 20 and 7M urea (Buffer A) and applied to a DEAE anion exchange column. The column was extensively washed in Buffer A, then bound protein was eluted using a 100-500 mM NaCl gradient in Buffer A. Peak fractions of gp41 activity were pooled and dialyzed to remove urea.

The p24 recombinant produced in E. coli (clone pB1) was purified by passage of bacterial lysate supernatant over an affinity column composed of Sepharose 4B beads bound with monoclonal anti-HIV-p24. The column was washed with a buffer containing 0.1% TRITON® X-100, and the bound p24 was eluted with the same buffer containing 4M guanidine hydrochloride (GuHCl). The eluted protein solution was dialyzed extensively, then reapplied to a second affinity column and eluted as described above. Peak fractions of p24 were pooled and dialyzed to remove GuHCl.

To further characterize the recombinant proteins, purified core or envelope antigens were subjected to SDS PAGE and Western Blot analysis according to Schupbach et al. , Science, 224, 503-505 (1984). After electrophoresis of purified envelope protein and staining of gels, specific bands of about 38 and 36 kD were detected along with a few bands of lower molecular weight. These two bands were strongly reactive with human polyclonal and mouse monoclonal antibodies against gp41. Amino terminal sequencing of these two bands demonstrated that both bands contain a portion of the carboxyl terminus of gp120 and a complete gp41 gene product.

The purified core antigen, after electrophoresis and gel staining migrated predominantly as a protein of about 28 kD along with some bands of lower molecular weight. The band was strongly reactive with human polyclonal and mouse monoclonal antibodies against p24. By amino terminal sequencing this band contained a portion of p17, entire p24 and a portion of p15 protein.

### EXAMPLE 3

### Competitive Immunoassay for Anti-HIV-gp41 and Anti-HIV-p24 in Human Sera

This is not an example according to the invention but is present for its content of information or techniques relevant to the present invention. Examples 4 to 6 are also not examples according to the invention.

This example demonstrates an immunoassay consisting of two solid phase detection systems for anti-HIV in a human biological sample. A first solid phase system was constructed according to the following procedure.

Six miillimeter (one-quarter inch) polystyrene beads were pre-coated with HIV antibodies purified from sera of AIDS patients using well known techniques.

The p24 protein described in Example 1 was attached to the precoated bead by exposing the beads to a solution containing an approximate 1:1000 dilution of p24 bacterial lysate which had been clarified by low speed (about 10,000 x g) centrifugation and diluted in 1% BSA. The beads were then incubated for two hours at room temperature, the solution removed, and the beads were washed and dried. The p24-coated beads were contacted with 200 ul HRPO labeled anti-HIV-p24 (0.2-0.5 ug/ml), a 50 ul serum sample from either an AIDS patient, an LAS patient or a normal control, and 20 ul of 0.5 M sodium citrate (pH 7.5). The mixture was incubated for 1 to 24 hours, preferably for 16 to 22 hours, at a temperature range of 15°C to 45°C. The beads were washed three times with 5 ml distilled water to remove unbound specimen and labeled antibody. The washed beads were contacted with 300 ul of o-phenylene-diamine hydrogen peroxide solution which forms a yellow-colored product in the presence of horseradish peroxidase. After incubation for approximately 30 minutes at room temperature, the addition of 1 ml of 1 N H₂SO₄ stopped the color formation. An approximately 50% reduction in color (absorbance 492 nm) of the sample when compared to a control (containing no anti-HIV) indicated a positive result.

The second solid phase system was constructed according to the procedure described above, by precoating beads with anti-HIV-gp41 followed by an overcoating of a bacterial lysate containing gp41 (approximately 1:50 dilution in 1% BSA). The gp41-coated beads were contacted with 200 ul HRPO-labeled anti-HIV gp41 (0.2-1.5 ug/ml), a 50 ul serum specimen from either an AIDS patient, an LAS patient or a normal control and 20 ul of 0.5M sodium citrate (pH 7.5). Incubation, color formation, and absorbance calculations were performed as described above.

In each detection system, the antibody of interest (serum antibody to HIV-p24 and serum antibody to HIV-gp41) competed with the corresponding labeled antibody (HRPO-labeled anti-HIV-p24 and HRPO-labeled anti-HIV-gp41) for a number of binding sites on the coated bead. Therefore, the intensity of color developed in each detection system was inversely related to the amount of anti-HIV-gp41 and anti-HIV-p24 in the specimen. A positive reaction in either detection system, or both, indicated a positive test for anti-HIV in the serum specimen.

When tested with the recombinant gp41 assay, 30 out of 30 AIDS patients' sera were positive, while none of 30 specimens from the normal control group were found positive. When tested with the recombinant p24 assay, 9 of the AIDS patients' sera were positive and 21 out of 29 ARC patients' sera tested positively.

### EXAMPLE 4

### Comparison of Example 3 Assay with Conventional Anti-HIV Screening Assay

Serum samples from patients with AIDS or lymphadenopathy syndrome (LAS), and normal subjects were tested using the assay of Example 3, (Assay A in Table 1), a commercially available enzyme immunoassay for anti-HIV, Abbott Laboratories, Abbott Park, Illinois, utilizing HIV antigen derived from cell culture (Assay B in Table 1), and the Western Blot procedure (Assay C in Table 1). The results shown in Table 1 indicate that all 91 of the AIDS patients' specimens reacted positively using the Assay A or Assay B procedures, while 90 out of 91 specimens reacted positively using the Western Blot procedure.

In the first detection system of the invention which detects antibodies to HIV p24, 10 of 41 samples from individuals with AIDS were positive, while all 91 samples were positive utilizing the second detection system of the invention which detects antibodies to HIV gp41. Since a positive reaction in either detection system, or both, indicates a positive result, all samples were defined as positive by the inventive assay procedure. All 62 samples from LAS patients were positive utilizing Assays A, B and C.

**TABLE 1**

| Comparison of Assays A, B and C (Positive/Total) | | | | |
|---|---|---|---|---|
| Samples | Assay A | | Assay B | Assay C |
| | Anti-p24 | Anti-gp41 | | |
| Normal Subjects | 0/375 | 0/375 | 0/375 | 0/375 |
| AIDS | 10/41 | 91/91 | 91/91 | 90/91 |
| LAS | 10/14 | 62/62 | 62/62 | 62/62 |

### EXAMPLE 5

### Example 3 Assay Compared to Western Blot

Samples of serum or plasma from blood donors which were repeatedly reactive in a commercially available enzyme immunoassay for anti-HIV, Abbott Laboratories, Abbott Park, Illinois, were tested with the present invention and by the Western Blot procedure. The results are shown in Table 2. Among the total of 351 repeatedly reactive specimens, 232 were negative using the Western Blot procedure and 232 were negative using the double detection system assay of Example 3. One sample which had an indeterminate reaction using Western Blot analysis, was positive in the Example 3 assay, being reactive in both detection systems. Of the 118 samples confirmed positive by the Western Blot procedure, all 118 were also positive utilizing the Example 3 assay, inasmuch as 3 samples which were negative in the anti-gp41 detection system were positive in the anti-p24 detection system.

**TABLE 2**

| Comparison of Western Blot Procedure to Example 3 Assay | | | |
|---|---|---|---|
| Samples (n=351) | Positive in Either or Both Detection Assays | Positive in Assay 1 (Anti-p24) | Positive in Assay 2 (Anti-gp41) |
| Western Blot negative (n=232) | 0/232 | 0/232 | 0/232 |
| Western Blot positive (n=118) | 118/118 | 102/118 | 115/118 |
| Western Blot undeterminable (n=1) | 1/1 | 1/1 | 1/1 |

The identification of biological samples which are positive for anti-HIV is important in the screening and protection of human blood supplies. These results show that the significant number of false positive specimens using the presently available screening assay could have been avoided using the present invention. This assay enables a more specific screening procedure that performs as well as the Western Blot for detecting true positive samples for anti-HIV without the problems of the Western Blot method such as labor-intensiveness and subjective interpretation.

### EXAMPLE 6

### Use of Example 3 Assay to Detect Serological Markers of Early Stage HIV Infection

Studies included two groups of hemophiliac patient populations in early stages of HIV infection. Group 1 consisted of 24 subjects who had tested seronegative for HIV infection, and group 2 consisted of 16 subjects who had previously tested seronegative and had converted to seropositive on the last given assay using a standard ELISA screening assay for detection of antibodies to HIV (Abbott Laboratories, Abbott Park, Illinois).

The patients were assayed at intervals of 1-6 months over a period of two years to assess the first detectable HIV markers and subsequent changes in the detectable markers over time. The specific tests for detectable markers included an assay for specific antibodies to HIV envelope protein, gp41, and to HIV core protein, p24, using the Example 3 assay, an assay for detection of HIV antigens (Abbott Laboratories, Abbott Park, Illinois) and a Western Blot for detection of antibodies to HIV.

The results indicated that before sero-conversion 14 of the 40 patients (35%) were positive for HIV antigens. In 6 cases, HIV antigens were the only detectable markers; in 3 additional antigen-positive cases which were negative for anti-HIV by standard ELISA method, anti-HIV could be detected by the Example 3 assay (anti-gp41) and by Western Blot method. This data indicates that the assay of Example 3 can be used to detect anti-HIV in early stages of HIV infection.

### EXAMPLE 7

### Immunoassay to Detect Anti-HIV-p24 and and Anti-HIV-p41 Using Labeled Anti-Human Antibody

This is not an example according to the invention but is present for its content of information or techniques relevant to the present invention.

This example demonstrates an immunoassay consisting of two solid phase detection systems for anti-HIV in a human biological sample. A first solid phase system was constructed according to the following procedure.

The purified p24 protein described in Example 2 was coated directly onto a six millimeter (one-quarter inch) polystyrene bead by exposing the beads to a solution containing about 0.1-0.5 ug/ml purified p24. The beads were incubated for 2 hours at 40°C and the solution removed. The beads were further incubated with 1% BSA solution, the solution removed, and the beads were washed and dried, The p24-coated beads were contacted with 200 ul of patient specimen diluted 1:40 in diluent containing goat serum, calf serum and E. coli (JM103) lysate. The mixture was incubated for 30 minutes to 1 hour, at 40°C. The beads were washed three times with 5 ml distilled water, then incubated for one-half to two hours at 40°C with 200 ul of affinity purified goat anti-human antibody conjugated to HRPO. The beads were washed again and contacted with 300 ul of OPD substrate solution as described in Example 3.

The second solid phase system was constructed according to the procedure described above, except that purified gp41 was coated directly on the bead at protein concentrations of 0.05 - 0.3 ug/ml. The anti-p41 assay protocol follows that described for anti-p24 above.

In each detection system, the antibody of interest (serum antibody to HIV-p24 and serum antibody to HIV-gp41) binds to the corresponding antigen coated bead. These bound antibodies are detected using goat anti-human antibody conjugate. Therefore, the intensity of color developed in each detection system was within limits directly related to the amount of anti-HIV-gp41 and anti-HIV-p24 in the specimen. A positive reaction in either detection system, or both, indicated a positive test for anti-HIV in the serum sample.

The above assay configuration may be modified by coating the p24 and p41 proteins onto the same solid support. In this modified assay, beads were simultaneously coated with both purified p24 and gp41 by exposing beads to a solution containing p24 at 0.1 - 0.5 ug/ml and gp41 at 0.05 - 0.3 ug/ml. Bead coating and assay procedures were, in all other respects, the same as that described above. Use of these beads allowed detection of both anti-gp41 and anti-p24 antibodies and gave significantly greater sensitivity than previous anti-HIV tests such as an ELISA anti-HIV screening test using whole viral lysate grown in HT-9 cells described above. (See Table 3.)

**TABLE 3**

| Sensitivity of Various Anti-HIV Assays: Reciprocal Endpoint Dilutions of Anti-HIV Positive Sera | | | |
|---|---|---|---|
| Serum Number | ELISA Anti-HIV | Example 7 Recombinant Based Anti-HIV Assay | Example 8 Recombinant Based Anti-HIV Sandwich |
| 1 | 128 | 1024 | 4096 |
| 2 | 32 | 512 | 2048 |
| 3 | 64 | 512 | 2048 |
| 4 | 256 | 2048 | 4096 |
| 5 | 128 | 1024 | 2048 |
| 6 | 512 | 4096 | 16384 |
| 7 | 512 | 4096 | 16384 |
| 8 | 1024 | 8192 | 37768 |
| 9 | 2048 | 4096 | 8192 |
| 10 | 32768 | 131072 | 262144 |
| 11 | 512 | 4096 | 16384 |

### EXAMPLE 8

### Sandwich Immunoassay to Detect Anti-HIV-p24 and Anti-HIV-gp4l Using Labeled Recombinant gp41 and p24

Beads coated with purified recombinant gp41 or p24 or both as described in Example 7 were used as the solid phase. The beads were contacted with a 200 ul serum sample and 20 ul of specimen addition solution containing nonionic detergent and E. coli JM103 lysate for 30 minutes to 24 hours at 15-40°C. Beads were washed with distilled water then contacted with 200 ul of solution containing HRPO-labeled antibodies to biotin and biotinylatedl purified p24 (for the anti-p24 assay), HRPO-labeled antibodies to biotin and biotinylated, purified gp41 (for the anti-gp41 assay) or HRPO-labeled antibodies to biotin and biotinylated, purified gp41 and p24 for the anti-HIV assay. Appropriate beads were incubated with corresponding conjugate solutions for 30 minutes to 2 hours at 40°C, then washed with distilled water and reacted with substrate as described in Example 3. Alternatively, the specimens and conjugate solutions may be added simultaneously to the bead, thereby eliminating one incubation and one wash step. In each detection system, the antibody or antibodies of interest (serum antibody to HIV-p24, serum antibody to HIV-gp41, or serum antibody to either gp41 or p24, or both) cross link antigen coated on the bead with the corresponding labeled antigen in the conjugate solution. A positive reaction in either the p24 or gp41 detection system, or both, or a positive reaction in the assay using p24 and gp41 on the same solid support, indicates a positive test for anti-HIV in the serum specimen. The sensitivity of the latter anti-HIV assay is shown in Table 3 and is significantly better than previous anti-HIV tests.

### EXAMPLE 9

### Immunoassay for Antibodies to HIV-p24 and p41

In another embodiment of the invention, two detection systems were utilized but in a different configuration. In the first detection system the solid support was coated with anti-HIV-p24, then exposed to the biological specimen and recombinant p24. After a washing step, the solid phase was contacted with labeled anti-HIV-p24 to detect the amount of bound p24. The absence of bound p24 indicated the presence of anti-HIV-p24 antibodies in the specimen.

In the second detection system the solid support was coated with anti-HIV-gp41, then exposed to the biological specimen and recombinant gp41. After a washing step, the solid phase was contacted with labeled anti-HIV-gp41 to detect the amount of bound gp41. The absence of bound gp41 indicated the presence of anti-HIV-gp41 antibodies in the specimen.

The assays of Example 3 and Example 9 were tested for specificity and sensitivity. To determine specificity a population of blood donors having no known risk factors for exposure to HIV were tested using both assays. The results are shown in Table 4. In the first detection system, i.e., the detection system for anti-p24, of both Examples 3 and 9, all the samples were negative for antibodies to p24. In the second detection system, i.e., the detection system for anti-gp41 all the samples were negative for antibodies to gp41.

**TABLE 4**

| Specificity of Examples 3 and 9 Assays (No. Positive/No. Negative) | | |
|---|---|---|
| FIRST DETECTION SYSTEM | | |
| No. Normal Samples | Example 3 | Example 9 |
| 160 | 0/160 | 0/160 |

| SECOND DETECTION SYSTEM | | |
|---|---|---|
| No. Normal Samples | Example 3 | Example 9 |
| 118 | 0/118 | 0/118 |

In order to determine sensitivity, a panel of eleven specimens from patients known to have antibodies to HIV by clinical history, Western Blot, and other screening assays was tested using serial dilutions of the specimens. The results in Tables 5 and 6 indicate that in the first detection system, only some patients had anti-p24. However, the Example 9 assay gave significantly higher endpoint dilutions for p24 antibodies, thus illustrating superior sensitivity. In the second detection system, all eleven samples were detected as positive in both the assays of Examples 3 and 9. However, the Example 9 assay gave significantly higher endpoint dilutions for gp41 antibodies, thus illustrating superior sensitivity.

**TABLE 5**

| Sensitivity of the First Detection System (anti-p24) | | | |
|---|---|---|---|
| Reciprocal Endpoint Dilutions of Patients Tested by Examples 3 and 9 Assays | | | |
| Patient | Diagnosis | Example 3 | Example 9 |
| 1 | AIDS | N | N |
| 2 | AIDS | ND | N |
| 3 | AIDS | ND | ND |
| 4 | AIDS | ND | 32 |
| 5 | AIDS | 64 | 1024 |
| 6 | ARC | ND | 2 |
| 7 | ARC | ND | 2 |
| 8 | ARC | ND | 4096 |
| 9 | ASYMP | 4096 | 8192 |
| 10 | ASYMP | 65,536 | 1,048,576 |
| 11 | ASYMP | ND | 512 |
| N=negative ND=not determinable | | | |

**TABLE 6**

| Sensitivity of the Second Detection System (Anti-gp41) | | | |
|---|---|---|---|
| Reciprocal Endpoint Dilutions of Patients Tested by Examples 3 and 9 Assays | | | |
| Patient | Diagnosis | Example 3 | Example 9 |
| 1 | AIDS | 2048 | 8192 |
| 2 | AIDS | 1024 | 4096 |
| 3 | AIDS | 512 | 1024 |
| 4 | AIDS | 4096 | 16,384 |
| 5 | AIDS | 2048 | 8192 |
| 6 | ARC | 8192 | 32,768 |
| 7 | ARC | 16,384 | 65,536 |
| 8 | ARC | 16,384 | 65,536 |
| 9 | ASYMP | 2048 | 16,384 |
| 10 | ASYMP | 32,768 | 131,768 |
| 11 | ASYMP | 8192 | 32,768 |

### EXAMPLE 10

### ENZYME IMMUNOASSAY FORMAT THAT ALLOWS EARLIER DETECTION OF HIV ANTIBODY BECAUSE OF INCREASED SENSITVITY TO DECAVALENT IMMUNOGLOBULIN M (IgM)

This example demonstrates an enzyme immunoassay format that allows for earlier detection of HIV antibody because of increased sensitivity to decavalent immunoglobulin M (IgM).

Patient. plasma sample was contracted with a polystyrene bead coated with recombinant gag ( p24) and env (gp41) proteins. After incubation at 40 C for 30 minutes the excess sample was removed by extensive washing with distilled water. The washed bead with bound HIV-1 antibodies was then contracted with a buffered solution containing p24 conjugated to horseradish peroxidase (HRPO), biotinylated gp41 and purified rabbit anti-biotin IgG conjugated to HRPO. After incubation at 40 C for 30 minutes excess conjugate was removed by extensive washing. Bound HRPO was detected by incubation with chromogenic substrate o-phenylenediamine and hydrogen peroxide for 30 minutes at room temperature. After 30 minutes the color reactor was stopped by the addition of 1M sulfuric acid. The resulting yellow orange color was quantitated by colorimetry at 492 nm and was compared with the negative control optical density obtained by substituting normal human plasma for patient sample in the assay. Samples with an OD492 greater than the negative control +0.1 were considered positive for HIV-1 antibodies.

Serial sample bleeds from several subjects undergoing seroconversion were assayed according to this assay format. These data are presented in Table 7 and compared to the ELISA anti-HIV screening test using whole viral lysate described above and the assay of Example 7. The anti-HIV-1 IgM and IgG levels, measured as described by Gaines ( H. Gaines et al. "Detection of Immunoglobulin M Antibody In Primary Human Immunodeficiency Virus Infection" Lancet i 1249-1253,1987) are also given in Table 7.

It is to be understood by those skilled in the art that portions or fragments of the p24 and gp41 proteins described herein, or substitution, deletion or addition analogs of these proteins, may be utilized in the assay methods of the invention.

Table 7 Comparison of Inventive Assay with Two Conventional EIAs for antibodies to HIV-I.

Assay results are give as the ratio between signal and cut-off (S/CO). Levels for IgM and IgG are give as signal to negative control (S/N). The first positive resutl (S/Co > 1.0) is marked *.

| donor ID | day relative to first sample | ELISA Anti-HIV | Example 7 Recombinant Based Anti-HIV Assay | Example 10 | IgM s/n | IgG s/n |
|---|---|---|---|---|---|---|
| 1 | 1 | 1.061* | 3.846* | 4.038* | 4.76 | 3.85 |
| | 6 | 1.591 | 4.564 | 4.406 | 4.23 | 4.20 |
| | 10 | 2.347 | 5.554 | 10.590 | 3.98 | 5.35 |
| | 16 | 3.680 | 5.779 | 14.115 | 3.39 | 7.00 |
| | 20 | 5.348 | 6.251 | 16.736 | 4.07 | 8.45 |
| 2 | 1 | 0.141 | 0.125 | 0.253 | 4.49 | 1.15 |
| | 6 | 0.242 | 0.911 | 9.655* | 4.83 | 1.20 |
| | 8 | 0.363 | 2.280* | 11.479 | 10.35 | 2.55 |
| | 14 | 0.960 | 4.003 | 5.824 | 16.50 | 6.35 |
| | 17 | 0.923 | 4.396 | 3.916 | 16.25 | 6.90 |
| | 21 | 2.008* | 4.925 | 5.149 | 13.50 | 8.10 |
| | 24 | 0.957 | 4.900 | 6.100 | 9.50 | 6.75 |
| 3 | 1 | 0.453 | 3.299* | 6.054* | 14.75 | 4.25 |
| | 5 | 1.252* | 4.507 | 7.019 | 20.60 | 8.30 |
| | 8 | 1.836 | 5.000 | 6.958 | 15.95 | 7.05 |
| 4 | 1 | 0.598 | 3.352* | 11.571* | 14.10 | 3.15 |
| | 5 | 0.786 | 3.274 | 10.452 | 11.15 | 2.80 |
| | 9 | 0.876 | 3.027 | 12.674 | 10.55 | 2.20 |
| | 12 | 1.185* | 4.232 | 11.318 | 8.25 | 2.45 |
| | 16 | 1.457 | 4.800 | 12.284 | 6.45 | 4.50 |
| | 19 | 1.783 | 5.197 | 13.241 | 5.50 | 5.60 |
| | 23 | 1.709 | 5.365 | 15.762 | 4.50 | 6.20 |
| | 26 | 2.417 | 6.444 | 14.820 | 3.80 | 7.05 |
| 5 | 1 | 0.494 | 2.570* | 9.456* | 10.05 | 3.40 |
| | 8 | 0.779 | 3.760 | 4.513 | 5.50 | 5.95 |
| | 10 | 0.829 | 4.339 | 5.441 | 5.45 | 6.25 |
| | 17 | 1.460* | 5.325 | 7.885 | 5.15 | 10.20 |
| | 22 | 1.994 | 5.454 | 8.743 | 3.70 | 12.50 |
| 6 | 1 | 4.296* | 6.926* | 13.157* | 5.95 | 17.40 |
| | 5 | 6.027 | 6.826 | 12.981 | 4.85 | 17.35 |
| 7 | 1 | 0.265 | 0.264 | 0.352 | 0.85 | 1.15 |
| | 3 | 0.201 | 0.229 | 0.268 | 1.25 | 1.00 |
| | 8 | 0.299 | 0.658 | 5.602* | 2.15 | 0.95 |
| | 10 | 0.457 | 1.701* | 12.352 | 6.25 | 1.40 |
| | 17 | 1.450* | 4.325 | 7.172 | 8.20 | 5.65 |
| | 20 | 1.682 | 5.018 | 8.375 | 7.55 | 7.75 |

## Claims

1. A method for detecting antibody to HIV in a biological sample employing a first and second solid support, the method comprising the steps of:
(a) contacting a first aliquot of a biological sample with a first solid support to which HIV-1 p24 recombinant protein has been attached;
(b) contacting the first solid support with labeled HIV-1 p24 recombinant protein;
(c) detecting the label to determine the presence of anti-HIV-1 p24 in the biological sample;
(d) contacting a second aliquot of the biological sample with a second solid support to which HIV-1 gp41 recombinant protein has been attached;
(e) contacting the second solid support with labeled HIV-1 gp41 recombinant protein;
(f) detecting the label to determine the presence of anti-HIV-1 gp41 in the biological sample;
wherein the presence of either anti-HIV-1 p24 or anti-HIV-1 gp41, or both, indicates the presence of anti-HIV in the biological sample.

2. The method of Claim 1 in which steps a and b are performed simultaneously and in which steps d and e are performed simultaneously.

3. A method for detecting antibody to HIV in a biological sample employing a solid support, the method comprising the steps of:
(a) contacting a biological sample with a solid support to which HIV-1 p24 recombinant protein and HIV-1 gp41 recombinant protein have been attached;
(b) contacting the solid support with labeled HIV-1 p24 recombinant protein and labeled HIV-1 gp41 recombinant protein;
(c) detecting the label to determine the presence of anti-HIV-1 p24 or anti-HIV-1 gp41, or both, in the biological sample;
wherein the presence of either anti-HIV-1 p24 or anti-HIV-1 gp41, or both, indicates the presence of anti-HIV in the biological sample.

4. The method of Claim 3 in which steps a and b are performed simultaneously.

5. A method for detecting antibody to HIV in a biological sample employing a first and second solid support, the method comprising the steps of:
(a) contacting a first aliquot of a biological sample with a first solid support to which HIV-1 p24 recombinant protein has been attached;
(b) contacting the first solid support with HIV-1 p24 recombinant protein conjugated to a first hapten;
(c) contacting the first solid support with labeled antibody to the first hapten;
(d) detecting the label to determine the presence of anti-HIV-1 p24 in the biological sample;
(e) contacting a second aliquot of the biological sample with a second solid support to which HIV-1 gp41 recombinant protein has been attached;
(f) contacting the second solid support with HIV-1 gp41 recombinant protein conjugated to a second hapten;
(g) contacting the second solid support with labeled antibody to the second hapten;
(h) detecting the label to determine the presence of anti-HIV-1 gp41 in the biological sample;
wherein the presence of either anti-HIV-1 p24 or anti-HIV-1 gp41, or both, indicates the presence of anti-HIV in the biological sample.

6. A method for detecting antibody to HIV in a biological sample employing a solid support, the method comprising the steps of:
(a) contacting a biological sample with a solid support to which HIV-1 p24 recombinant protein and HIV-1 gp41 recombinant protein have been attached;
(b) contacting the solid support with HIV-1 p24 recombinant protein conjugated to a first hapten and HIV-1 gp41 recombinant protein conjugated to a second hapten;
(c) contacting the solid support with labeled antibody to the first and second haptens;
(d) detecting the label to determine the presence of anti-HIV-1 p24 or anti-HIV-1 gp41, or both, in the biological sample;
wherein the presence of either anti-HIV-1 p24 or anti-HIV-1 gp41, or both, indicates the presence of anti-HIV in the biological sample.

## Patentansprüche

1. Verfahren zum Nachweis von Antikörpern gegen HIV in einer biologischen Probe, das einen ersten und einen zweiten festen Träger verwendet, wobei das Verfahren die folgenden Schritte umfaßt:
(a) In-Kontakt-Bringen eines ersten aliquoten Anteils einer biologischen Probe mit einem ersten festen Träger, an dem rekombinantes HIV-1-p24-Protein verankert ist;
(b) In-Kontakt-Bringen des ersten festen Trägers mit markiertem rekombinantem HIV-1-p24-Protein;
(c) Nachweisen der Markierung, um die Anwesenheit von anti-HIV-1-p24 in der biologischen Probe zu bestimmen;
(d) In-Kontakt-Bringen eines zweiten aliquoten Anteils der biologischen Probe mit einem zweiten festen Träger, an dem rekombinantes HIV-1-gp41 verankert ist;
(e) In-Kontakt-Bringen des zweiten festen Trägers mit markiertem rekombinantem HIV-1-gp41-Protein;
(f) Nachweisen der Markierung, um die Anwesenheit von anti-HIV-1-gp41 in der biologischen Probe zu bestimmen;
wobei die Anwesenheit von anti-HIV-1-p24 oder von anti-HIV-1-gp41 oder von beiden die Anwesenheit von anti-HIV in der biologischen Probe anzeigt.

2. Verfahren nach Anspruch 1, bei dem die Schritte a und b gleichzeitig durchgeführt werden und bei dem die Schritte d und e gleichzeitig durchgeführt werden.

3. Verfahren zum Nachweis von Antikörpern gegen HIV in einer biologischen Probe, das einen festen Träger verwendet, wobei das Verfahren folgende Schritte umfaßt:
(a) In-Kontakt-Bringen einer biologischen Probe mit einem festen Träger, an dem rekombinantes HIV-1-p24-Protein und rekombinantes HIV-1-gp41-Protein verankert sind;
(b) In-Kontakt-Bringen des festen Trägers mit markiertem rekombinantem HIV-1-p24-Protein und mit markiertem rekombinantem HIV-1-gp41-Protein;
(c) Nachweisen der Markierung, um die Anwesenheit von anti-HIV-1-p24 oder anti-HIV-gp41 oder von beiden in der biologischen Probe zu bestimmen;
wobei die Anwesenheit von anti-HIV-1-p24 oder von anti-HIV-1-gp41 oder von beiden die Anwesenheit von anti-HIV in der biologischen Probe anzeigt.

4. Verfahren nach Anspruch 3, bei dem die Schritte a und b gleichzeitig durchgeführt werden.

5. Verfahren zum Nachweis von Antikörpern gegen HIV in einer biologischen Probe, das einen ersten und einen zweiten festen Träger verwendet, wobei das Verfahren folgende Schritte umfaßt:
(a) In-Kontakt-Bringen eines ersten aliquoten Anteils einer biologischen Probe mit einem ersten festen Träger, an dem rekombinantes HIV-1-p24-Protein verankert ist;
(b) In-Kontakt-Bringen des ersten festen Trägers mit rekombinantem HIV-1-p24-Protein, das an ein erstes Hapten konjugiert ist;
(c) In-Kontakt-Bringen des ersten festen Trägers mit markierten Antikörpern gegen das erste Hapten;
(d) Nachweisen der Markierung, um die Anwesenheit von anti-HIV-1-p24 in der biologischen Probe zu bestimmen;
(e) In-Kontakt-Bringen eines zweiten aliquoten Anteils der biologischen Probe mit einem zweiten festen Träger, an dem rekombinantes HIV-1-gp41-Protein verankert ist;
(f) In-Kontakt-Bringen des zweiten festen Trägers mit rekombinantem HIV-1-gp41-Protein, das an ein zweites Hapten konjugiert ist;
(g) In-Kontakt-Bringen des zweiten festen Trägers mit markierten Antikörpern gegen das zweite Hapten;
(h) Nachweisen der Markierung, um die Anwesenheit von anti-HIV-1-gp41 in der biologischen Probe zu bestimmen;
wobei die Anwesenheit von anti-HIV-1-p24 oder von anti-HIV-1-gp41 oder von beiden die Anwesenheit von anti-HIV in der biologischen Probe anzeigt.

6. Verfahren zum Nachweis von Antikörpern gegen HIV in einer biologischen Probe, das einen festen Träger verwendet, wobei das Verfahren folgende Schritte umfaßt:
(a) In-Kontakt-Bringen einer biologischen Probe mit einem festen Träger, an dem rekombinantes HIV-1-p24-Protein und rekombinantes HIV-1-gp41-Protein verankert sind;
(b) In-Kontakt-Bringen des festen Trägers mit rekombinantem HIV-1-p24-Protein, das an ein erstes Hapten konjugiert ist und mit rekombinantem HIV-1-gp41-Protein, das an ein zweites Hapten konjugiert ist;
(c) In-Kontakt-Bringen des festen Trägers mit markierten Antikörpern gegen das erste und das zweite Hapten;
(d) Nachweisen der Markierung, um die Anwesenheit von anti-HIV-1-p24 oder anti-HIV-1-gp41 oder von beiden in der biologischen Probe zu bestimmen;
wobei die Anwesenheit von anti-HIV-1-p24 oder von anti-HIV-1-gp41 oder von beiden die Anwesenheit von anti-HIV in der biologischen Probe anzeigt.

## Revendications

1. Procédé de détection d'anticorps anti-VIH dans un échantillon biologique, utilisant un premier et un deuxième support solide, le procédé comprenant les étapes consistant à
(a) mettre une première quantité aliquote d'un échantillon biologique en contact avec un premier support solide auquel a été fixée de la protéine recombinée p24 de VIH-1;
(b) mettre le premier support solide en contact avec de la protéine recombinée p24 de VIH-1 marquée ;
(c) détecter le marqueur pour déterminer la présence d'anticorps anti-p24 de VIH-1 dans l'échantillon biologique ;
(d) mettre une deuxième quantité aliquote de l'échantillon biologique en contact avec un deuxième support solide auquel a été fixée de la protéine recombinée gp41 de VIH-1 ;
(e) mettre le deuxième support solide en contact avec de la protéine recombinée gp41 de VIH-1 marquée ;
(f) détecter le marqueur pour déterminer la présence d'anticorps anti-gp41 de VIH-1 dans l'échantillon biologique ;
étant entendu que la présence d'anticorps anti-p24 de VIH-1 et/ou d'anticorps anti-gp41 de VIH-1 indique la présence d'anticorps anti-VIH dans l'échantillon biologique.

2. Procédé selon la revendication 1, dans lequel les étapes (a) et (b) sont mises en oeuvre simultanément et dans lequel les étapes (d) et (e) sont mises en oeuvre simultanément.

3. Procédé de dosage d'anticorps anti-VIH dans un échantillon biologique, utilisant un support solide, le procédé comprenant les étapes consistant à :
(a) mettre un échantillon biologique en contact avec un support solide auquel ont été fixées de la protéine recombinée p24 de VIH-1 et de la protéine recombinée gp41 de VIH-1 ;
(b) mettre le support solide en contact avec de la protéine recombinée p24 de VIH-1 marquée et de la protéine recombinée gp41 de VIH-1 marquée ;
(c) détecter le marqueur pour déterminer la présence d'anticorps anti-p24 de VIH-1 et/ou d'anticorps anti-gp41 de VIH-1 dans l'échantillon biologique;
étant entendu que la présence d'anticorps anti-p24 de VIH-1 et/ou d'anticorps anti-gp41 de VIH-1 indique la présence d'anticorps anti-VIH dans l'échantillon biologique.

4. Procédé selon la revendication 3, dans lequel les étapes (a) et (b) sont mises en oeuvre simultanément.

5. Procédé de détection d'anticorps anti-VIH dans un échantillon biologique, utilisant un premier et un deuxième support solide, le procédé comprenant les étapes consistant à :
(a) mettre une première quantité aliquote d'un échantillon biologique en contact avec un premier support solide auquel a été fixée de la protéine recombinée p24 de VIH-1 ;
(b) mettre le premier support solide en contact avec de la protéine recombinée p24 de VIH-1, conjuguée à un premier haptène ;
(c) mettre le premier support solide en contact avec un anticorps marqué dirigé contre le premier haptène ;
(d) détecter le marqueur pour déterminer la présence d'un anticorps anti-p24 de VIH-1 dans l'échantillon biologique ;
(e) mettre une deuxième quantité aliquote de l'échantillon biologique en contact avec un deuxième support solide auquel a été fixée de la protéine recombinée gp41 de VIH-1 ;
(f) mettre le deuxième support solide en contact avec la protéine recombinée gp41 de VIH-1, conjuguée à un deuxième haptène ;
(g) mettre le deuxième support solide en contact avec un anticorps marqué dirigé contre le deuxième haptène ;
(h) détecter le marqueur pour déterminer la présence d'anticorps anti-gp41 de VIH-1 dans l'échantillon biologique ;
étant entendu que la présence d'anticorps anti-p24 de VIH-1 et/ou d'anticorps anti-gp41 de VIH-1 indique la présence d'anticorps anti-VIH dans l'échantillon biologique.

6. Procédé de détection d'anticorps anti-VIH dans un échantillon biologique, utilisant un support solide, le procédé comprenant les étapes consistant à:
(a) mettre un échantillon biologique en contact avec un support solide auquel ont été fixées de la protéine recombinée p24 de VIH-1 et de la protéine recombinée gp41 de VIH-1 ;
(b) mettre le support solide en contact avec de la protéine recombinée p24 de VIH-1, conjuguée à un premier haptène, et de la protéine recombinée gp41 de VIH-1, conjuguée à un deuxième haptène ;
(c) mettre le support solide en contact avec un anticorps marqué dirigé contre le premier et le deuxième haptène ;
(d) détecter le marqueur pour déterminer la présence d'anticorps anti-p24 de VIH-1 et/ou d'anticorps anti-gp41 de VIH-1 dans l'échantillon biologique ;
étant entendu que la présence d'anticorps anti-p24 de VIH-1 et/ou d'anticorps anti-gp41 de VIH-1 indique la présence d'anticorps anti-VIH dans l'échantillon biologique.
